(19) Europäisches Patentamt

European Patent Office

Office européen des brevets

(11) **EP 1 100 481 B1**

(12) **EUROPEAN PATENT SPECIFICATION**

(45) Date of publication and mention
of the grant of the patent:
**25.05.2005  Bulletin 2005/21**

(21) Application number: **99940025.2**

(22) Date of filing: **20.07.1999**

(51) Int Cl.[7]: **A61K 31/00**, A61P 1/00,
A61K 9/02, A61K 9/32

(86) International application number:
**PCT/EP1999/005160**

(87) International publication number:
**WO 2000/006132 (10.02.2000 Gazette 2000/06)**

(54) **PHARMACEUTICAL FORMULATIONS COMPRISING BECLOMETHASONE DIPROPIONATE FOR THE TREATMENT OF INFLAMMATORY BOWEL DISEASE**

PHARMAZEUTISCHE ZUSAMMENSETZUNG ENTHALTEND BECLOMETHASON-DIPROPIONAT ZUR BEHANDLUNG ENTZÜNDLICHER DARMERKRANKUNGEN

FORMULATIONS PHARMACEUTIQUES SERVANT A TRAITER LA MALADIE INTESTINALE INFLAMMATOIRE

(84) Designated Contracting States:
**AT BE CH CY DE DK ES FI FR GB GR IE IT LI LU
MC NL PT SE**
Designated Extension States:
**AL LT LV MK**

(30) Priority: **28.07.1998  IT  MI981741**

(43) Date of publication of application:
**23.05.2001  Bulletin 2001/21**

(60) Divisional application:
**04020016.4 / 1 486 209**

(73) Proprietor: **CHIESI FARMACEUTICI S.p.A.
I-43100 Parma (IT)**

(72) Inventors:
• **CHIESI, Paolo
I-43100 Parma (IT)**
• **MUSA, Rossella
I-43100 Parma (IT)**
• **BERNINI, Eva
I-43100 Parma (IT)**
• **CASTIGLIONE, Gian, Nicola
I-43100 Parma (IT)**

(74) Representative: **Minoja, Fabrizio, Dr.
Bianchetti Bracco Minoja S.r.l.
Via Plinio, 63
20129 Milano (IT)**

(56) References cited:
**EP-A- 0 301 423          EP-A- 0 375 063
EP-A- 0 517 274          WO-A-92/16206
WO-A-96/03115          WO-A-97/25980
WO-A-99/16454**

• **VIGNOTTI D. ET AL: "Topical treatment of active distal ulcerative colitis with beclomethasone dipropionate" CURRENT THERAPEUTIC RESEARCH, vol. 52, no. 5, 1992, pages 659-665, XP002119773 cited in the application**
• **STEED, K. P. ET AL: "The in vivo behavior of a colonic delivery system: a pilot stud in man" INT. J. PHARM. (1994), 112(3), 199-206 , XP002128287**

Remarks:
The file contains technical information submitted after the application was filed and not included in this specification

**Description**

The invention relates to pharmaceutical compositions for oral administration to be used for the treatment of Inflammatory Bowel Disease (IBD).

The invention more particularly relates to the use of a topically active corticosteroid, i.e. beclomethasone dipropionate, in the following also referred to as BDP, for the preparation of pharmaceutical compositions for the treatment of Inflammatory Bowel Diseases such as Ulcerative Colitis (UC) and Crohn's Disease (CD).

IBD is an inflammatory disorder of unknown cause which is characterized by a chronic relapsing - remitting course, in which relapses and remissions alternate. IBD's have increased in incidence, in particular in the Northern areas of Western Countries.

UC is characterized by an inflammatory process confined to the most superficial layer of the intestinal wall (submucosa is saved) primarily involving the colonic mucosa. One of the most frequent localizations is the rectal ampulla from which the inflammatory process can extend to the colon, but not beyond the ileo-cecal valve.

CD is yet another type of inflammatory disease of unknown etiology which mainly affects the distal ileum, but which may occur in any part of the bowel. Contrary to what is observed in UC, the inflammatory process is of "segmental" type (affected portions alternate to health areas) and it affects all intestinal layers.

The medical treatment of said diseases is based on the use of immunosuppressive and anti-inflammatory drugs.

The most effective treatment relies on the use by oral route of corticosteroids such as prednisolone, as they possess both immunosuppressive and antiinflammatory properties. However, their use is limited due to the possible systemic side effects (such as oedema, hypertension) and inhibitory effects on adrenocorticotropic hormone (ACTH) with consequent reduction of cortisol production by the adrenal glands. In order to avoid such consequences, the advised treatment provides for an initial high doses for a few weeks, followed by the gradual reduction to maintenance dosage, which should however still be the minimal one able of controlling the clinical symptoms.

Corticosteroids with high topical activity but low systemic bioavailability have been therefore developed; said drugs maintain an effectiveness comparable to that of the systemic ones, even though they show a reduced absorption and therefore a marked decrease in the above mentioned adverse effects

By virtue of their selective action on the affected intestinal mucosa and the high degree of first-pass metabolism in the liver, with a resulting reduction of the systemic absorption, these novel topical corticosteroids have high therapeutical effectiveness with poor incidence of both systemic and endocrine side effects.

The effectiveness of topical corticosteroids, administered in the form of enemas, in distal UC has been widely demonstrated and more recently such treatment has been also proved to be useful in patients affected by CD.

Beclomethasone dipropionate is a potent, well tolerated topical corticosteroid which has successfully been used in the treatment of distal UC. Although being safe and effective, it has only been used in the form of galenic or extemporarily compounded preparations, as no ready-to-use formulations of suitable pharmaceutical characteristics have been commercially made available so far.

The present invention relates to ready-to-use pharmaceutical formulations for the administration of beclomethasone dipropionate (BDP) at the intestinal site, for use in the treatment of IBD.

The invention provides a formulation for distal ileum and proximal colon delivery in the form of modified-release tablets formed by a central core which allows sustained release of the active ingredient (BDP) coated with a polymeric gastroresistant coating.

In order to meet the pharmacological need for a local antiinflammatory action, a tablet for the treatment of inflammatory bowel disease should be able of delivering the active ingredient as near as possible to the action site.

The ideal approach is the specific delivery of the active ingredient to the colon. The formulations for colonic delivery should be designed to withstand both low and slightly basic pH values for several hours. During this time they are assumed to pass the stomach and the small intestine and to reach the large intestine where the coat disintegrates and the drug release process is initiated. The polymers used for this purpose are commonly acrylic acid derivatives or cellulose derivatives. More difficult to predict is the location, and hence the environment, in which the coat starts to degrade. Depending on the gastrointestinal mobility pattern, it can occur deep in the colon, as well as halfway down the small intestine. In addition, the presence of short-chain fatty acids, carbon dioxide, and other fermentation products, often reduces the pH of the ascending colon and call in question the use of the pH just to trigger drug release. Therefore the problem cannot be solved by using only a particular gastro-resistant polymeric coating as, in many cases, a significant amount of the active ingredient could be released prior to the arrival at the targeted site.

In the prior art different approaches are reported in order to target as much drug as possible to the desired location (i.e colon) where it should exert its therapeutic activity.

WO 9116881, WO 9200732 e WO 97/27843 all claim different kind of pharmaceutical compositions characterised in that the coating and/or the matrix is a substance e.g a polysaccharide such as pectin or its derivatives which is specifically attacked by intestinal bacteria.

EP 0521074 claims a special osmotic device whose enteric coat is hydrophobic for preventing the flux of water through said coating. The corresponding formulations are of complicated design and manufacture.

Furthermore, it is well known that the ionic strength of the medium could affect the properties of some of the hydrophobic excipients used at this purpose and, hence, the disintegration rate of the tablets. The gastric-intestinal fluids of the patients at which said formulations are addressed to, are expected to be poor in salts and, hence, of low ionic strength; accordingly the choice of the excipients and the corresponding ratios should be evaluated in view of such potential drawbacks.

A simple solution is provided by the modified-release tablet of the invention which has the following characterizing features:

1) a gastro-resistant coating, made of a film of methacrylic acid copolymer in aqueous phase;
2) a sustained-release core, obtained by using hydroxypropylmethyl cellulose, in concentration ranging from 15 to 35% by weight on the core weight;
3) a ratio between water soluble and insoluble excipients of 5:3 w/w where the amount of soluble excipients does not exceed 60% by weight of the weight of the tablet.

It has been surprisingly found that the choice of the excipients and in particular the ratio between the water soluble and insoluble excipients is of paramount importance for controlling the disintegration of the tablets and, hence, for triggering the release of the drug in fluids of low ionic strength. The compositions of the inventions are stable, simple to make and allow most of the dose to be released at the targeted site (distal ileum and proximal colon).

Other hydrophobic excipients for controlling the release of the drug such as hydroxypropyl cellulose and methyl cellulose and other polymers resistant up to pH 5.5, such as cellulose phthalate derivatives, for coating the tablets can be advantageously used.

The preparation of the formulations is illustrated in greater detail in the following examples which are not meant to be limiting the invention.

Example 1. Preparation of BDP modified-release tablets

Unitary composition

Ingredients:

| Micronized beclomethasone dipropionate | 5.0 mg |
|---|---|
| Spray dried lactose F.U. | 48.4 mg |
| Microcrystalline cellulose (Avicel®PH 102) | 8.0 mg |
| Maize starch | 3.0 mg |
| Hydroxypropyl methyl cellulose 2208 (Methocel® K4M) | 20.0 mg |
| Magnesium stearate | 0.6 mg |
| | 85.0 mg |

Gastro-resistant coating:

Film made of methacrylic acid copolymer (Eudragit ® L 30 D).

First, the core is prepared as follows. BDP is dispersed in half the amount of spray dried lactose. All the components are sieved through a 25 mesh sieve, the BDP/lactose premix is mixed with the other components and compressed. Finally, cores are coated.

Example 2. Disintegration and release characteristics of the tablets

Disintegration.

Disintegration parameters were evaluated according to the Official Pharmacopoeia (F.U.).

According to F.U., gastric resistant tablets should show no signs of disgregation or fracture upon immersion at pH 1.2 for 2 hours, and should disintegrate within a time of 60' at most, at pH 6.8.

The disintegration rate of the tablets of the invention was also checked in buffer solutions with different ionic strengths.

In particular, the solutions at different pH and ionic strength reported as follows were used:

| Solutions and relevant pH | | Ionic strength (I) |
|---|---|---|
| 1 | Phosphate buffer solution F.U. pH 6.8 | 0.6 |
| 2 | Phosphate buffer solution F.U. pH 6.8 | 0.4 |
| 3 | Phosphate buffer solution IDMA pH 6.9 | 0.1 |
| 4 | Phosphate buffer solution pH 6.8 | 0.1 |
| 5 | 0.1 N HCl solution | 0.1 |
| 6 | 0.1 N HCl solution + 0.9% NaCl | 0.2 |
| 7 | 0.1 N HCl solution + 9% NaCl | 1.6 |

The corresponding disintegration time of the sustained-release core of the tablets of the invention, are reported as follows:

| Solution | Time (min) |
|---|---|
| Solution 1 | 5 |
| Solution 2 | 5 |
| Solution 3 | 60 |
| Solution 4 | 150 |
| Solution 5 | 150 |
| Solution 6 | >60 |
| Solution 7 | 6 |

A similar behavior was observed for the tablets with gastro-resistant coating when tested at pH 6.8 - 6.9.

As it can be inferred from the obtained results, the tablets are significantly affected by the different ionic strengths. Said test is of paramount importance in order to predict the *in vivo* disintegration time of the tablets, as the formulation is addressed to patients whose salt concentration in the intestinal fluids is probably reduced.

F.U. advises indeed a medium for carrying out the test whose salt concentration is much higher than that present in the body; therefore the *in vivo* disintegration behavior of the tablets of the invention is more likely to correspond to that observed in buffers 3 and 4; in said buffers, the ionic strength is lower as it has been adjusted for better simulate the conditions of the intestinal fluids of a patient affected by IBD..

The disintegration behavior of the tablets was also evaluated *in vivo*, at the intestinal site, in a study carried out on 8 healthy volunteers. A scintigraphic technique was employed as it is suitable for evaluating the position and integrity of the tablet within the gastrointestinal tract (Steed K.P. et al. Int J Pharmaceutics 1994; 112:199-206). The results confirmed that the tablet does not disintegrate in the stomach: the scintigraphic images evidence that onset of the erosion occurs in the small intestine and in the proximal colon, gradually continuing in the intestinal transit, so that most of the dose can reach the distal intestine. These findings confirmed that the modified-release tablets of the invention meet the requirements considered necessary for a formulation designed for the topical treatment of inflammatory bowel disease.

Release

In order to evaluate the release characteristics of the active ingredient from the tablets, a test was carried out using the apparatus for the determination of the disintegration time of tablets, reported in F.U. IX edition, vol. I, page 403, according to the conditions reported in USPXX/NFXV page 959 (apparatus n. 3 for the dissolution test).

The tablets were immersed in media with pH varying in progression from 1.5 to 7.2. The test was carried out for eight hours.

As the active ingredient is insoluble in water, it was considered 'dissolved' once it was present as dispersed particles into the medium. Said particles were subsequently dissolved in ethanol for the spectrophotometric determination. To avoid interferences, the analyses were carried out with comparison to a blank. Solutions with increasing pH: 1.5 - 4.5 - 6.9 - 7.2, in a 900 ml volume, were used. Four tablets were placed in the basket of the apparatus and 900 ml of the medium at pH 1.5 pre-heated to 37°C±0.5°C, were added; the vessel was kept under stirring for one hour. The test liquid was discarded and replaced with 900 ml of the medium at pH = 4.5, operating under the same conditions.

The test was then continued with the medium at pH = 6.9, keeping the vessel under stirring for two hours and finally with the medium at pH = 7.2, under stirring for four hours. Samples (10 ml) were withdrawn after 1, 2,4,6 and 8 hours.

The results are reported as follows:

| Sample | pH | Partial time | Total time | % release |
|--------|-----|--------------|------------|-----------|
| 1 | 1.5 | 1 hour | 1 hour | 0 |
| 2 | 4.5 | 1 hour | 2 hours | 0.9 |
| 3 | 6.9 | 2 hours | 4 hours | 36.0 |
| 4 | 7.2 | 2 hours | 6 hours | 32.4 |
| 5 | 7.2 | 2 hours | 8 hours | 31.2 |
| | | | | 100.5 |

Example 3. Evaluation of the absorption.

In order to demonstrate that no systemic absorption occurred, BDP and its major active metabolites, Beclomethasone-17-monopropionate (B 17MP) and Beclomethasone (BOH), were determined in plasma and urine samples in subjects undergoing treatment with the formulation of the invention.

A first study was performed in 9 healthy male volunteers, after a single administration of a gastro-resistant modified-release tablet by oral route. No detectable amounts of the drug and its metabolites were found on plasma and urine samples, indicating that, upon oral administration, the BDP activity is exerted only topically.

A subsequent study carried out in 10 ileostomized patients, 5 of whom treated with 5 mg modified-release BDP coated tablets and the other 5 with 5 mg BDP uncoated tablets, confirmed the absence of the active ingredient and of its metabolites both in plasma and urine samples collected during the study. The analysis of intestinal effluates showed the presence of significant amounts of BDP and B17MP, indicating that a significant release of active ingredient occurred at the action site, i.e. the distal part of the ileum. A higher release was observed for the gastro-resistant formulation

The absence of systemic absorption of unmetabolised BDP and/or of its metabolites was also assessed in a pharmacokinetic-scintigraphic study performed in healthy volunteers (example 4, § "Disintegration"), to further confirm the topical action of the compound.

**Claims**

1.  A pharmaceutical formulation for the treatment of inflammatory bowel disease in the form of a gastro-resistant modified-release tablet formed by an outer polymeric coating resistant up to pH 5.5 and a central core, said formulation containing: i) beclomethasone dipropionate as active ingredient; ii) an hydrophobic excipient for controlling the release of the active ingredient in concentrations ranging from 15 to 35% by weight on the core weight; and being **characterized by** a ratio between water soluble and insoluble excipients of 5:3 w/w where the amount of soluble excipients does not exceed 60% by weight on the weight of the tablet.

2.  Pharmaceutical formulation according to claim 1, wherein the enteric coating is made of methacrylic acid or cellulose phthalate polymers, preferably Eudragit®L 30 D.

3.  Pharmaceutical formulation according to claim 1, wherein the hydrophobic excipient is a hydroxypropylmethyl cellulose, hydroxypropyl cellulose or methyl cellulose, to provide the gradual and controlled release of the active ingredient in fluids at basic pH and low ionic strength.

4.  Pharmaceutical formulation according to claim 1, wherein the hydrophobic excipient is hydroxypropylmethyl cellulose.

5.  Pharmaceutical formulation according to claim 4, further containing microcrystalline cellulose.

6.  Pharmaceutical formulation according to claim 4, further containing maize starch.

**Patentansprüche**

1. Pharmazeutische Formulierung zur Behandlung von entzündlicher Darmerkrankung in Form einer magenresistenten Tablette mit modifizierter Freisetzung, die aus einer äußeren polymeren Beschichtung, die bis zu pH 5,5 resistent ist, und einem zentralen Kern besteht, wobei die Formulierung enthält: (i) Beclomethasondipropionat als aktives Ingredienz; (ii) ein hydrophobes Exzipienz zur Kontrolle der Freisetzung des aktiven Ingredienz in Konzentrationen im Bereich von 15 bis 35 Gew.-%, bezogen auf das Kerngewicht; **dadurch gekennzeichnet, dass** das Verhältnis zwischen wasserlöslichen und -unlöslichen Exzipienzien 5:3 G/G ist, wobei die Menge an löslichen Exzipienzien 60 Gew.-%, bezogen auf das Gewicht der Tablette, nicht übersteigt.

2. Formulierung nach Anspruch 1, wobei die magensaftresistente Beschichtung aus Methacrylsäure- oder Cellulosephthalatpolymeren, vorzugsweise Eudragit®L 30 D, besteht.

3. Pharmazeutische Formulierung nach Anspruch 1, wobei das hydrophobe Exzipienz eine Hydroxypropylmethylcellulose, Hydroxypropylcellulose oder Methylcellulose ist, um die allmähliche und kontrollierte Freisetzung des aktiven Ingredienz in Flüssigkeiten mit basischem pH und niedriger Ionenstärke bereitzustellen.

4. Pharmazeutische Formulierung nach Anspruch 1, wobei das hydrophobe Exzipienz Hydroxypropylmethylcellulose ist.

5. Pharmazeutische Formulierung nach Anspruch 4, die außerdem mikrokristalline Cellulose enthält.

6. Pharmazeutische Formulierung nach Anspruch 4, die außerdem Maisstärke enthält.

**Revendications**

1. Une formulation pharmaceutique pour le traitement d'une infection intestinale inflammatoire sous la forme d'un comprimé gastrorésistant à libération modifiée formé d'un enrobage polymère externe résistant jusqu'à pH 5,5 et d'un noyau central, ladite formulation contenant : i) du dipropionate de béclométasone comme ingrédient actif ; ii) un excipient hydrophobe pour régler la libération de l'ingrédient actif, à des concentrations comprises entre 15 et 35 % en poids par rapport au poids du noyau ; et étant **caractérisée par** un rapport entre les excipients solubles et insolubles dans l'eau de 5:3 en poids, la quantité d'excipients solubles ne dépassant pas 60 % en poids par rapport au poids du comprimé.

2. Formulation pharmaceutique selon la revendication 1, dans laquelle l'enrobage entérique est formé de polymères d'acide méthacrylique ou de phtalate de cellulose, de préférence Eudragit® L 30 D.

3. Formulation pharmaceutique selon la revendication 1, dans laquelle l'excipient hydrophobe est l'hydroxypropylméthylcellulose, l'hydroxypropylcellulose ou la méthylcellulose, pour réaliser la libération progressive et réglée de l'ingrédient actif dans les fluides à pH basique et à faible force ionique.

4. Formulation pharmaceutique selon la revendication 1, dans laquelle l'excipient hydrophobe est l'hydroxypropylméthylcellulose.

5. Formulation pharmaceutique selon la revendication 4, contenant, de plus, de la cellulose microcristalline.

6. Formulation pharmaceutique selon la revendication 4, contenant, de plus, de l'amidon de maïs.